(19) Europäisches Patentamt
European Patent Office
Office européen des brevets

(11) Publication number: **0 088 423**
**B1**

(12) **EUROPEAN PATENT SPECIFICATION**

(45) Date of publication of patent specification: **03.06.87**

(21) Application number: **83102236.3**

(22) Date of filing: **07.03.83**

(51) Int. Cl.$^4$: **C 07 C 49/167,**
C 07 C 49/185, C 07 C 49/497,
C 07 C 49/687, C 07 C 45/67,
C 07 C 45/66, C 07 C 69/716,
C 07 C 67/347

(54) **Fluoroketones and cyclic condensation products thereof.**

(30) Priority: **08.03.82 JP 36127/82**

(43) Date of publication of application:
**14.09.83 Bulletin 83/37**

(45) Publication of the grant of the patent:
**03.06.87 Bulletin 87/23**

(84) Designated Contracting States:
**CH DE FR GB LI**

(56) References cited:
**US-A-2 706 739**
**US-A-3 277 147**

(73) Proprietor: **Daikin Kogyo Co., Ltd.**
**Shinhankyu Building No 1-12-39, Umeda Kita-ku**
**Osaka-shi Osaka-fu (JP)**

(72) Inventor: **Ishikawa, Nobuo, Prof. Dr.**
**10-10, Shinoharadai-machi Kohoku-ku**
**Yokohama-shi Kanagawa-ken (JP)**
Inventor: **Kitazume, Tomoya, Dr.**
**24-1-115, Kitasenzoku 3-chome**
**Ota-ku Tokyo (JP)**

(74) Representative: **Patentanwälte TER MEER - MÜLLER - STEINMEISTER**
**Mauerkircherstrasse 45**
**D-8000 München 80 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 088 423 B1

**Description**

Field of the Invention

The present invention relates to fluoroketones and cyclic condensation products thereof.

Description of the Prior Art

It is known that introduction of fluorine atoms into organic compounds often gives fluoro compounds having specific biological activities. In a series of organic syntheses making use of the characteristic chemical properties of polyfluoroolefins particularly, the present authors studied site-selective introduction of a fluorine atom by use of fluoroacetoacetate. As a result, they discovered new useful organic compounds embodying the present invention.

Objects and Summary of the Invention

Accordingly, an object of the present invention is to provide fluoroketones having biological activities.

Another object of the invention is to provide cyclic condensation products of the fluoroketones that are useful, for example, as intermediates in the synthesis of some medicines.

The present invention relates to fluoroketones characterized by the general formula:

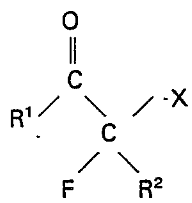

wherein $R^1$ is a hydrogen atom or an alkyl group having ten carbon atoms at maximum, $R^2$ is a hydrogen atom, an aliphatic hydrocarbonoxycarbonyl group having eleven carbon atoms at maximum or an aromatic hydrocarbonoxycarbonyl group of eleven carbon atoms at maximum having a benzene ring linked through an ether linkage, which benzene ring may have a butyl or lower alkyl group as its substituent and X is a group that is derived from an unsaturated compound that is an acceptor in the Michael reaction as expressed by either of the following two general formulas:

$$YCH{=}CHR^3$$

wherein $R^3$ is a hydrogen atom or an alkyl group having ten carbon atoms at maximum, Y is formyl, an alkylcarbonyl group having eleven carbon atoms at maximum, an alkoxycarbonyl group having eleven carbon atoms at maximum or a cyano group, and

$$YC{=}CR^3$$

wherein $R^3$ and Y are as defined above.

A further subject matter of the present invention are cyclic condensation products of the above fluoroketones, which are characterized by either of the general formulas:

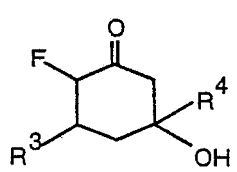

and

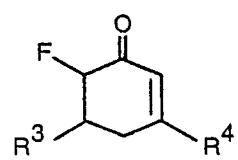

wherein $R^3$ and $R^4$ are independently hydrogen atoms, or alkyl groups having ten carbon atoms at maximum and $R^4$ in addition may be an alkoxy group having ten carbon atoms at maximum.

Description of the Preferred Examples

In the above general formula of fluoroketones embodying the present invention, $R^1$ may be an alkyl group having ten carbon atoms at maximum, for example, methyl, ethyl, propyl, isopropyl, or butyl group and $R^2$ may be a hydrogen atom, an aliphatic hydrocarbonoxycarbonyl group having eleven carbon atoms at maximum, for example, a methyl ester, ethyl ester, propyl ester, or isopropyl ester group. In addition, $R^2$ may be an aromatic hydrocarbonoxycarbonyl group of eleven carbon atoms at maximum, having a benzene ring linked through an ether linkage. Such benzene ring may have a butyl or lower alkyl group as its substituent.

Further, X is a group derived from an unsaturated compound that is an acceptor in the Michael reaction as expressed by either of following two general formulas:

$$YCH{=}CHR^3$$

wherein $R^3$ is a hydrogen atom, an alkyl group having ten carbon atoms at maximum, Y is formyl, an alkylcarbonyl group having eleven carbon atoms at maximum, an alkoxycarbonyl group having eleven carbon atoms at maximum or a cyano group, and

$$YC{\equiv}CR^3$$

wherein $R^3$ and Y are as defined above.

Further, the aforementioned cyclic condensation products embodying the present invention are derivatives of the above fluoroketones. $R^3$ and $R^4$ in the general formula of the former are thus provided just by incorporating the $R^3$ and $R^4$ of the fluoroketone of the invention, namely, 2-fluorodiketone as they are. In addition, this $R^3$ is the same as in the above acceptor of the Michael Reaction. These groups $R^3$ and $R^4$ may be independently a group having ten carbon atoms at maximum, for example, a methyl, ethyl, or propyl group, while $R^4$ may be an alkoxy group having ten carbon atoms at maximum, for example, one derived from methyl alcohol, ethyl alcohol, or propyl alcohol. It is noted that in the above fluorodiketone that is cyclized to the condensation product thereof, $R^2$ may be one of the aliphatic hydrocarbonoxycarbonyl groups of eleven carbon atoms at maximum as mentioned above or an aromatic hydrocarbonoxycarbonyl group of eleven carbon atoms at maximum having a benzene ring linked through an ether linkage.

To illustrate the present invention more fully, the introduction of fluorine atom with use of 2-fluoroacetoacetate is described below by way of example. The manner how the fluorine atom can be introduced into other 2-fluoroketones and their cyclic condensation products will be understood similarly.

First, 2-fluoroacetoacetate that is considered a building block of monofluoro compound can be synthesized in a high yield from trifluoroethane according to the following reaction:

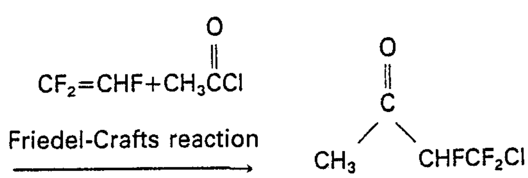


1

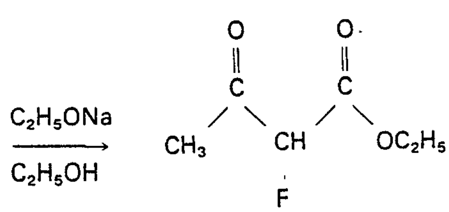


2

Namely, the starting material or trifluoroethene undergoes the Friedel-Crafts reaction to produce 4-chloro-3,4,4-trifluoro-2-butanone *1,* which is then converted under the action of a base to 2-fluoroacetoacetate *2.* For the above reaction itself, a reaction process already proposed by the present authors in "Bull. Chem. Soc. Jpn" 54, 832 (1981) is applicable. To describe more specifically, the reaction step from *1 to 2* in the above reaction process proceeds as follows:

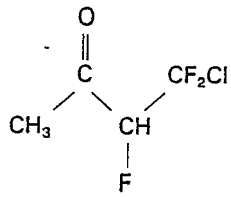

*1*

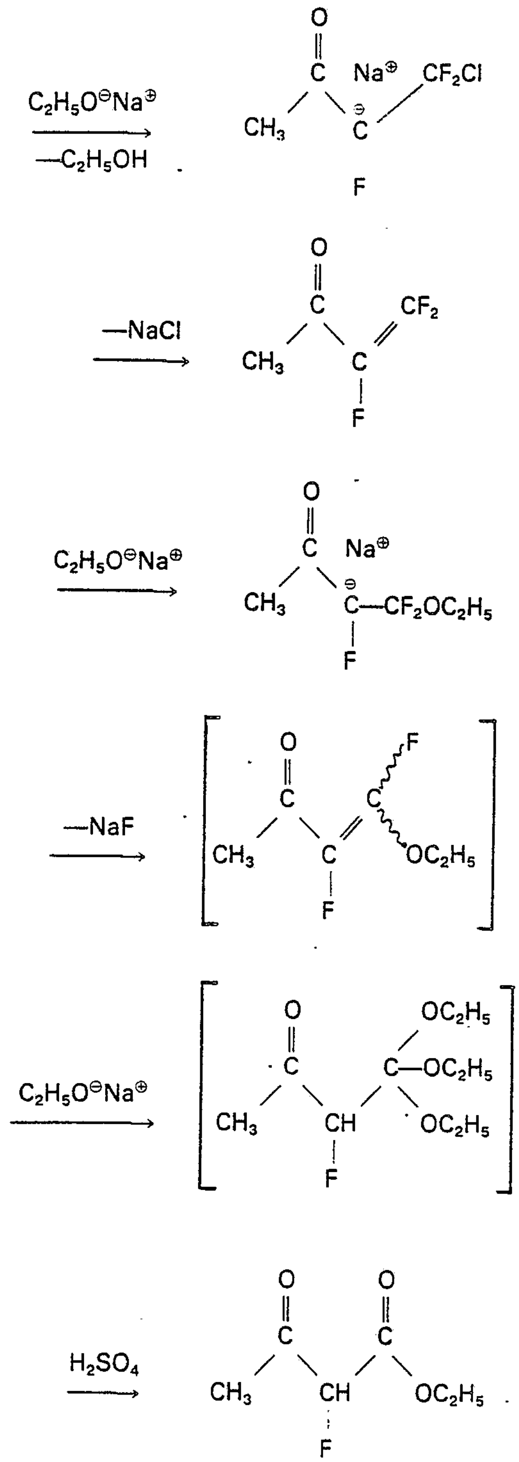

$C_2H_5O^{\ominus}Na^{\oplus}$
$-C_2H_5OH$
$-NaCl$
$C_2H_5O^{\ominus}Na^{\oplus}$
$-NaF$
$C_2H_5O^{\ominus}Na^{\oplus}$
$H_2SO_4$


*2*

Next, this 2-fluoroacetoacetate *2* is converted to the corresponding addition product by the Michael reaction under the action of a base. For the base used here, spray-dried potassium fluoride that is a useful source of free fluoride ions ($F^-$) is preferably used. Such spray-dried potassium fluoride may be prepared, for example, by spraying a commercially available aqueous solution of potassium fluoride of concentration of 30 weight-percent at a rate of 3 kg/h in hot air current of 300 to 500°C for drying. The physical properties of the spray-dried KF are a grain size of 10 to 50 µm, specific surface area of 1.3 $m^2$/g, bulk density of 0.3 to 0.7 g/ml, and moisture content of 0.24%. The spray-dried KF thus has a remarkably large specific surface area as compared to preparations prepared by other methods, freeze-drying and roast-drying. It is a powerful fluorinating agent that gives a fluorinated compound at a high yield in short time. The above spray-dried potassium fluoride is an excellent reagent that releases the fluoride ion as a useful base. As shown in the following reaction formula, the released free fluoride ion reacts with 2-fluoroacetoacetate *2* to selectively produce an anion *3*, which is added to the acceptor *4* in the Michael reaction. The Michael addition product *5* is thus readily made available.

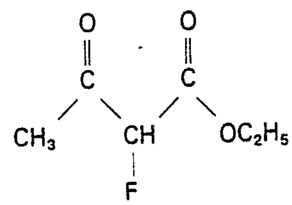

*2*

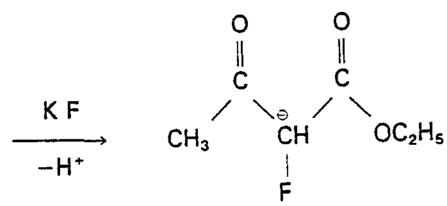


*3*

*3* +

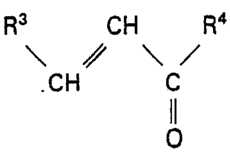

*4*

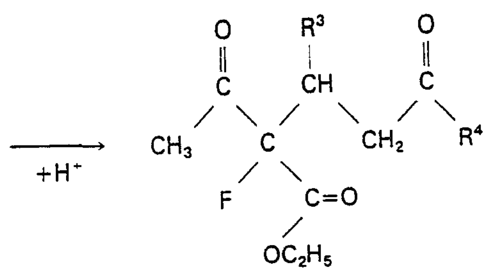


*5*

In the above Michael reaction, various acceptors were used to produce corresponding addition products as listed in the next table. The table shows that acceptors whose electronegative group is the carbonyl group give addition products in higher yields. Each asterisk in the table indicates that the addition product was produced in a form of mixture, which was separated by gas chromatography.

| Acceptor | Michael addition product | Yield (%) | Reaction time (h) | B.P. °C/mbar (°C/mmHg) |
|---|---|---|---|---|
| 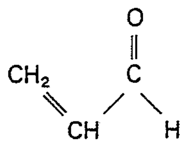 | 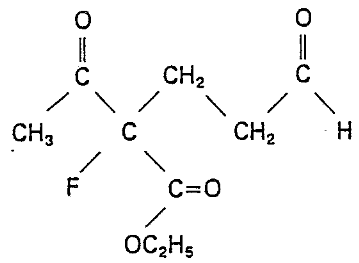 | >99 | 1 | 94~96/0.93 (94~96/0.7) |
| 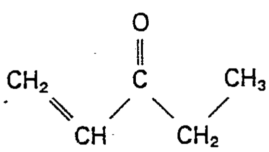 | 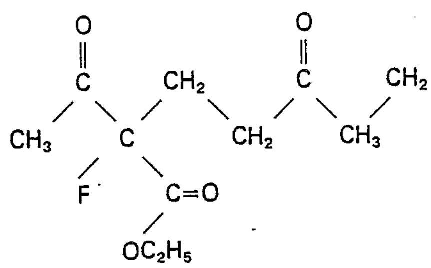 | >99 | 1 | 104~106/1.07 (104~106/08) |
| 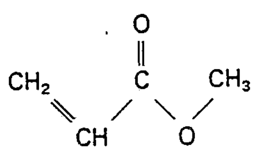 | 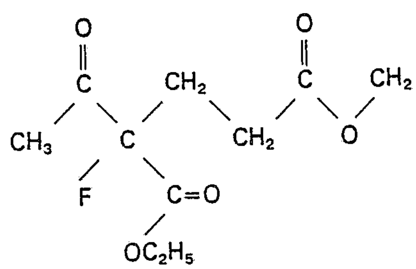 | 84 | 1 | 96~97/1.19 (96~97/09 |
| 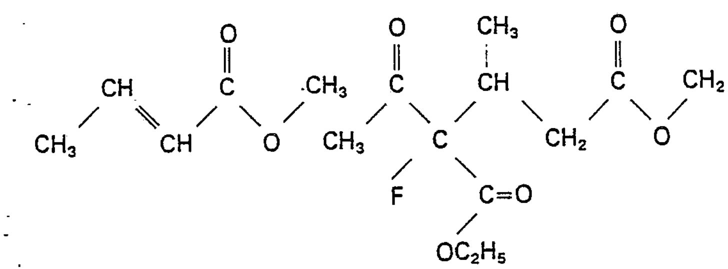 | | 14 | 24 | * |
| 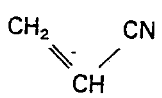 | 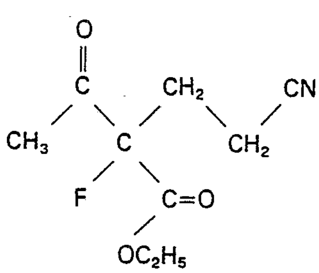 | 43 | 1 | * |

Particularly, the Michael addition product *5* was expected to produce the corresponding monofluorodiketone by decarboxylation. The authors thus tried its decarboxylation by various methods. As a result, it was found that use of hydrochloric acid gives the intended monofluorodiketone in a very high yield by the following reaction:

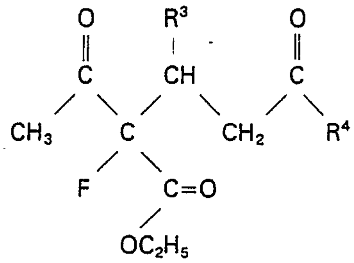

5

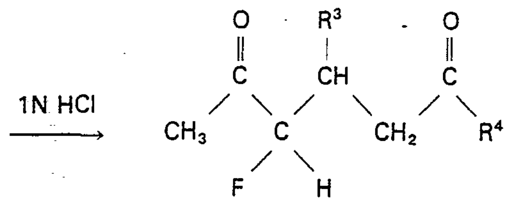


6

The diketone *6* undergoes intramolecular cyclization or the Claisen condensation according to the reaction formula as shown below to change to a cyclic condensation product *7* or derivative of cyclohexanone, which is further dehydrated to a derivative of cyclohexanone *8*.

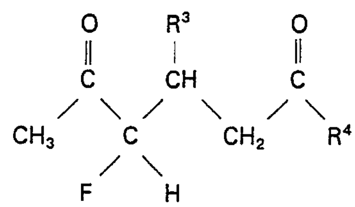

6

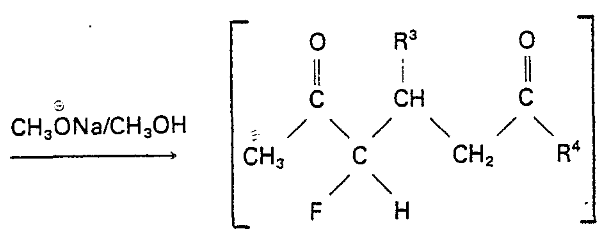


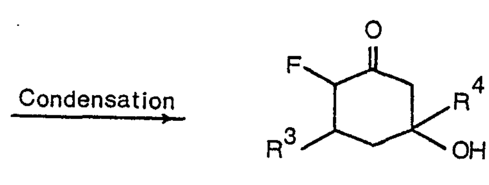


7

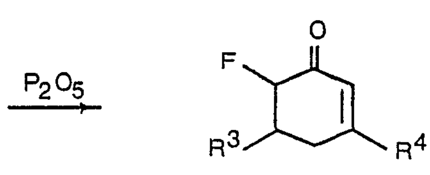


8

As mentioned above, by using, for example, the starting material, trifluoroethene as a building block in incorporation of the fluorine atom, there can be produced a fluoroketone and its cyclic condensation product of the present invention with the fluorine atom introduced site-selectively.

The invention will be understood more fully with reference to the following examples. However, these examples are intended to illustrate the invention and are not to be construed to limit the scope of the invention. Various modifications and variations will thus be possible within the wording of the claims.

Example 1

Synthesis of $CH_3COCF(CO_2C_2H_5)CH_2CH_2COCH_3$ (5):

1.48 g (10 mmol) of 2-fluoroacetoacetate, 0.84 g (12 mmol) of methyl vinyl ketone and 1.74 g (30 mmol) of spray-dried potassium fluoride were added to 20 ml of sulfolane and the mixture was agitated 1 hr at room temperature. 500 ml of water was added to the reaction solution and the oily layer was extracted therefrom with diethyl ether. The extracts were dried with magnesium sulfate.

After evaporation of solvent, the residues were distilled under reduced pressure, when a product of formula *5* was obtained (Yield: >99%, B.P.: 94 to 96°C/0.93 mbar (0.7 mmHg)).

Mass and NMR spectroscopy with this product gave the following results:

Mass: $m^+$ 218

NMR: $^1H(\delta, CDCl_3)$ 1.3 ($CH_2CH_3$), 2.3 ($CH_3CO$), 2.1 ($CH_3CO$), 2.3—3.1 ($CH_2CH_2$), 4.3 ($CH_3CH_2$)
$^{19}F(\delta)$ 88(CF) (External standard: $CF_3CO_2H$)

Example 2

Synthesis of $CH_3COCF(CO_2C_2H_5)CH_2CH_2COCH_2CH_3$ (5):

1.48 g (10 mmol) of 2-fluoroacetoacetate, 1.0 g (10 mmol) of ethyl vinyl ketone and 1.74 g (30 mmol) of spray-dried potassium fluoride were added to 20 ml of sulfolane and the mixture was agitated 1 hr at room temperature. 500 ml of water was added to the reaction solution and the oily layer was extracted therefrom with diethyl ether. The extracts were dried with magnesium sulfate.

After evaporation of solvent, the residues were distilled under reduced pressure, when another product of formula *5* was obtained (Yield: >99%, B.P.: 104 to 106°C/1.07 mbar/0.8 mmHg)).

Mass and NMR spectroscopy with this product gave the following results:

Mass: $M^+$ 232

NMR: $^1H(\delta, CDCl_3)$ 1.3 ($CH_3CH_2$), 2.2 ($CH_3CO$), 3.6 ($CH_3$), 1.0 ($CH_3CH_2$), 2.1—2.7 (6H), 4.3 ($CH_3CH_2$)
$^{19}F(\delta)$ 88.1(CF) (External standard: $CF_3CO_2H$)

Example 3

Synthesis of $CH_3COCHFCH_2CH_2COCH_3$ (6):

2.18 g (10 mmol) of an addition product of formula (5) $CH_3COCF(CO_2Et)CH_2CH_2COCH_3$ and 2 ml of 1N hydrochloric acid were added to 10 ml of diethyl ether and the mixture was agitated 24 hr at room temperature. The oily layer was separated and subjected to distillation under reduced pressure to produce a product of formula *6* $CH_3COCHFCH_2CH_2COCH_3$ (Yield: 65%, B.P.: 75—76°C/5.33 mbar(4 mmHg)).

Mass and NMR spectroscopy with this product gave the following results:

Mass: $M^+$ 146

NMR: $^1H(\delta, CDCl_3)$ 4.5 (1H), 1.0 ($CH_3$), 2.0—2.8, 2.1 ($CH_3CO$),
$^{19}F(\delta)$ 113(CF) (External standard: $CF_3CO_2H$)

Example 4

Synthesis of $CH_3COCHFCH_2CH_2COCH_2CH_3$ (6):

2.32 g (10 mmol) of another addition product of formula (5) $CH_3COCF(CO_2Et)CH_2CH_2COCH_2CH_3$ and 2 ml of 1N hydrochloric acid were added to 10 ml of diethyl ether and the mixture was agitated 24 hr at room temperature. The oily layer was separated and subjected to distillation under reduced pressure to produce a product of formula *6* $CH_3COCHFCH_2CH_2COCH_2CH_3$ (Yield: 70%, B.P.: 80—82°C/4.0 mbar/3 mmHg)).

Mass and NMR spectroscopy with this product gave the following results:

Mass: $M^+$ 160

NMR: $^1H(\delta, CDCl_3)$ 4.6 (1H), 1.0 (3H) 2.1 (3H), 2.0—2.6 (6H)
$^{19}F(\delta)$ 112.5 (External standard: $CF_3CO_2H$)

Example 5

Synthesis of a compound *7*,

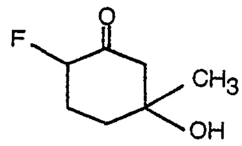

The mixture of 1.46 g (10 mmol) of diketone $CH_3COCHFCH_2CH_2COCH_3$ and 20 ml of sodium methoxide was subjected 5 hr to the reaction at 50°C. 500 ml of water was then added to the mixture and the oily layer was extracted therefrom with diethyl ether. Extracts were dried with magnesium sulfate and then the solvent was evaporated for removal. Distillation under reduced pressure resulted in a product *7* (Yield: 52%, B.P.: 76—79°C/2.67 mbar(2 mmHg)).

Mass and NMR spectroscopy with this product gave the following results:

Mass: $M^+$ 146
NMR: $^1H(\delta, CDCl_3)$ 1.0 (3H), 2.0—3.1 (6H) 4.5 (1H), 5.1 (1H)
$^{19}F(\delta)$ 113 (External standard: $CF_3CO_2H$)

Example 6

Synthesis of a compound *7*,

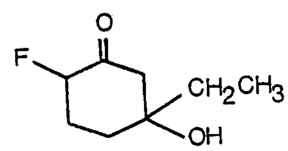

Using 1.60 g (10 mmol) of diketone *6* $CH_3COCHFCH_2CH_2COCH_2CH_3$ and 20 ml of sodium methoxide, a similar reaction to Example 5 was performed. Distillation under reduced pressure resulted in a product *7* (Yield: 56%, B.P.: 83—86°C/2.67 mbar(2 mmHg)).

Mass and NMR spectroscopy with this product gave the following results:
Mass: $M^+$ 160
NMR: $^1H(\delta, CDCl_3)$ 1.0—1.2 (6H), 2.0—3.3 (6H) 4.5 (1H), 5.2 (1H)
$^{19}F(\delta)$ 112 (External standard: $CF_3CO_2H$)

Example 7

Synthesis of a compound *8*,

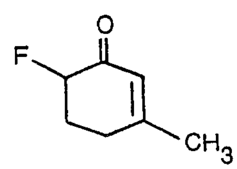

With addition of 2 g of phosphorus pentoxide, 1.46 g (10 mmol) of compound *6* was dehydrated at 150°C. The product was purified by distillation. The compound *8* was thus obtained (Yield: 63%, B.P.: 90—93°C/16.0 mbar/(12 mmHg)).

Mass and NMR spectroscopy with this product gave the following results:

Mass: $M^+$ 128
NMR: $^1H(\delta, CDCl_3)$ 2.1 (3H), 2.0—3.0 (4H) 4.6 (1H), 5.2 (1H)
$^{19}F(\delta)$ 113.5 (External standard: $CF_3CO_2H$)

Example 8

Synthesis of a compound 8,

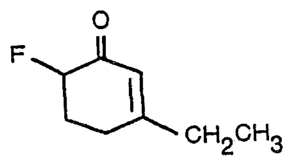

With addition of 2 g of phosphorus pentoxide, 1.6 g (10 mmol) of compound *7* was dehydrated at 150°C. The product was purified by distillation. The compound *8* was thus obtained (Yield: 52%, B.P.: 83—84°C/8.0 mbar(6 mmHg)).

Mass and NMR spectroscopy with this product gave the following results:

Mass: $M^+$ 142

NMR; $^1H(\delta, CDCl_3)$, 1.0 (3H), 2.0—3.1 (6H) 4.6 (1H), 5.2 (1H)

$^{19}F(\delta)$ 114 (External standard: $CF_3CO_2H$)

**Claims**

1. Fluoroketones characterized by the general formula:

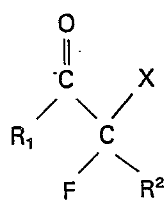

wherein $R^1$ is a hydrogen atom or an alkyl group having ten carbon atoms at maximum, $R^2$ is a hydrogen atom, an aliphatic hydrocarbonoxycarbonyl group having eleven carbon atoms at maximum or an aromatic hydrocarbonoxycarbonyl group of eleven carbon atoms at maximum having a benzene ring linked through an ether linkage, which benzene ring may have a butyl or lower alkyl group as its substituent and X is a group that is derived from an unsaturated compound that is an acceptor in the Michael reaction as expressed by either of the following two general formulas

$$YCH{=}CHR^3$$

wherein $R^3$ is a hydrogen atom or an alkyl group having ten carbon atoms at maximum, Y is formyl, an alkylcarbonyl group having eleven carbon atoms at maximum, an alkoxycarbonyl group having eleven carbon atoms at maximum or a cyano group, and

$$YC{\equiv}CR^3$$

wherein $R^3$ and Y are as defined above.

2. Cyclic condensation products of said fluoroketones of claim 1, characterized by either of the following general formulas:

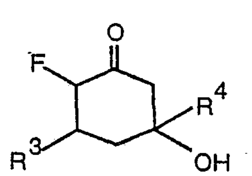

and

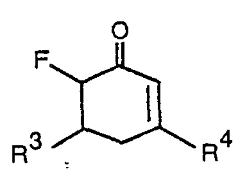

wherein $R^3$ and $R^4$ are independently hydrogen atoms, or alkyl groups having ten carbon atoms at maximum and $R^4$ in addition may be an alkoxy group having ten carbon atoms at maximum.

**Patentansprüche**

1. Fluorketone der allgemeinen Formel

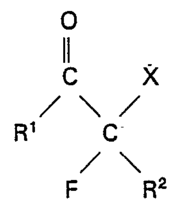

in der $R^1$ ein Wasserstoffatom oder eine Alkylgruppe mit höchstens 10 Kohlenstoffatomen, $R^2$ ein Wasserstoffatom, eine aliphatische Kohlenwasserstoffoxycarbonylgruppe mit höchstens 11 Kohlenstoffatomen oder eine aromatische Kohlenwasserstoffoxycarbonylgruppe mit höchstens 11 Kohlenstoffatomen und einem durch eine Etherbindung verbundenen Benzolring, wobei der Benzolring durch Butyl- oder niedrige Alkylgruppen substituiert sein kann, bedeuten und X eine Gruppe bedeutet, welche aus einer ungesättigten Verbindung, die einen Akzeptor bei der Michael-Reaktion darstellt, entstanden ist und einer der beiden folgenden allgemeinen Formeln entspricht:

$$YCH{=}CHR^3$$

worin $R^3$ ein Wasserstoffatom oder eine Alkylgruppe mit höchstens 10 Kohlenstoffatomen, Y eine Formylgruppe, eine Alkylcarbonylgruppe mit höchstens 11 Kohlenstoffatomen, eine Alkoxycarbonylgruppe mit höchstens 11 Kohlenstoffatomen oder eine Cyanogruppe bedeuten und

$$YC{\equiv}CR^3$$

worin $R^3$ und Y die oben angegebenen Bedeutungen aufweisen.

2. Cyclische Kondensationsprodukte der Fluorketone nach Anspruch 1, gekennzeichnet durch eine der folgenden allgemeinen Formeln:

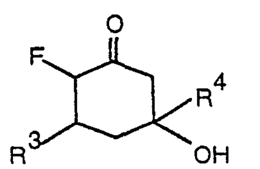

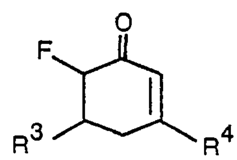

worin $R^3$ und $R^4$ voneinander unabhängig Wasserstoffatome oder Alkylgruppen mit höchstens 10 Kohlenstoffatomen bedeuten und $R^4$ zusätzlich eine Alkoxygruppe mit höchstens 10 Kohlenstoffatomen bedeuten kann.

**Revendications**

1. Fluorocétones, caractérisées par la formule générale:

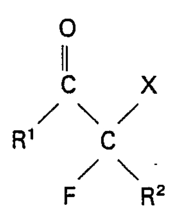

dans laquelle $R^1$ est un atome d'hydrogène ou un groupe alkyle ayant 10 atomes de carbone au maximum, $R^2$ est un atome d'hydrogène, un groupe (hydrocarbylealiphatique)-oxycarbonyle ayant 11 atomes de carbone au maximum ou un groupe (hydrocarbylearomatique)-oxycarbonyle de 11 atomes de carbone au maximum et comportant un cycle benzénique lié par l'intermédiaire d'une liaison éther, ce cycle benzénique pouvant comporter comme substituant un groupe butyle ou un groupe alkyle inférieur et X est un groupe qui est dérivé d'un composé insaturé qui est un accepteur dans la réaction de Michael tel qu'il est défini par l'une ou l'autre des deux formules générales suivantes:

$$YCH{=}CHR^3$$

dans laquelle $R^3$ est un atome d'hydrogène ou un groupe alkyle ayant 10 atomes de carbone au maximum, Y est un groupe formyle ou alkylcarbonyle ayant 11 atomes de carbone au maximum, un groupe alkoxycarbonyle ayant 11 atomes de carbone au maximum ou un groupe cyano, et

$$YC{\equiv}CR^3$$

dans laquelle $R^3$ et Y sont tels que définis ci-dessus.

2. Produits cycliques de condensation desdites fluorocétones de la revendication 1, caractérisé par l'une ou l'autre des formules générales suivantes:

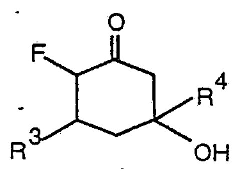

und

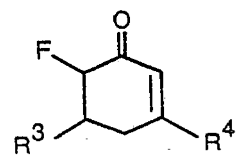

dans lesquelles $R^3$ et $R^4$ sont indépendamment des atomes d'hydrogène, des groupes alkyle ayant 10 atomes de carbone au maximum et $R^4$ peut être en outre un groupe alkoxy ayant 10 atomes de carbone au maximum.